# FASCICULE DE BREVET EUROPEEN

(11) **EP 2 318 191 B1**
(45) Date de publication et mention de la délivrance du brevet: **19.04.2017**
(21) Numéro de dépôt: 09737058.9
(22) Date de dépôt: 15.07.2009
(51) Int. Cl.: B29C 39/00, A61J 1/03, A61K 9/70, B65B 3/06, B65D 75/36

(54) **PROCÉDÉ DE FABRICATION DE FILMS DIRECTEMENT EN ALVÉOLE**
VERFAHREN ZUR HERSTELLUNG VON FILMEN DIREKT IN BLISTERVERPACKUNGEN
METHOD FOR PRODUCING FILMS DIRECTLY IN BLISTER TRAYS

(30) Priorité: 16.07.2008 FR 0854852
(43) Date de publication de la demande: 11.05.2011
(73) Titulaire: Roquette Frères, 62136 Lestrem (FR)
(72) Inventeur: FRANCOIS, Alain, F-62232 Hinges (FR); LEFEVRE, Philippe, F-59660 Haverskerque (FR); SIMON, Denis, F-59495 Lefrinckoucke (FR)
(74) Mandataire: Cabinet Plasseraud
(86) Numéro de dépôt international: PCT/FR2009/051402
(87) Numéro de publication internationale: WO 2010/007312

(56) Documents cités:
- WO-A-00/42992
- FR-A1- 2 781 667
- JP-A- 2004 231 516
- US-A- 5 369 937
- US-A1- 2004 076 799

## Description

La présente invention porte sur un procédé d'obtention d'un film susceptible d'être applicable notamment dans l'industrie alimentaire ou pharmaceutique. De plus, un support alvéolaire spécialement destiné à la mise en oeuvre dudit procédé est décrit.

Le procédé selon l'invention convient particulièrement bien pour la préparation de films contenant des principes actifs ou des films aromatiques. Ces films peuvent être envisagés avec un support, tel est le cas des patchs ou sans support dans le cas par exemple des feuilles d'arôme. Les feuilles d'arôme sont de très fines feuilles qui, suivant l'application souhaitée, peuvent être placées sur la langue dans le cas de films oraux, et fondent instantanément tout en diffusant un arôme, par exemple de la menthe. Les films peuvent convenir également pour des applications pharmaceutiques ne nécessitant pas spécifiquement une fonte instantanée du film, on peut citer par exemple la libération retardée d'actifs ou la mucoadhésion.

Il est connu de l'homme du métier des industries pharmaceutiques ou alimentaires, un procédé d'obtention de films oraux issu de technologies dérivées des bandes magnétiques pour les vidéos notamment.

Les procédés de l'art antérieur comprennent de manière générale les étapes suivantes :
- une première étape permettant l'obtention d'une bobine de film à partir d'un mélange filmogène,
- une seconde étape au cours de laquelle la bobine est découpée aux dimensions souhaitées et
- une troisième étape de conditionnement des films obtenus.

La première étape consiste en l'étalement dudit mélange en une large bande à la surface d'un film de plastique servant de support. Elle est suivie d'une phase de séchage de la fine couche de mélange par un flux d'air. La phase de séchage nécessite un contrôle et un traitement de l'air pulsé sur la bande de film, que ce soit dans le cadre de films destinés à une application alimentaire ou pharmaceutique. L'air pulsé peut, dans certains cas, nécessiter un traitement avant d'être rejeté dans l'environnement. Tel est le cas notamment d'une utilisation de produits toxiques pour l'homme ou l'environnement. En effet, l'air peut s'être chargé de substances toxiques du fait du passage en phase gaz d'additifs volatils issus du mélange ou de microparticules émises par les projections séchées de mélange ou par la bande de film obtenue.

Au terme de cette première étape, la bande de film et son support sont enroulés de sorte à obtenir une bobine de film. Plus rarement et suivant les caractéristiques de plasticité du film, la bande de film peut être obtenue et enroulée en bobine sans support. Cependant, l'absence de support accentue le risque de rupture de la bande.

Lors d'une seconde étape, la bobine de film est déroulée et la bande de film est séparée de son support afin d'être découpée suivant la forme et la taille désirées. Cette étape est une source de beaucoup de pertes puisque suivant les coupes effectuées, toute la largeur de la bande n'est pas nécessairement utilisée. Ainsi, avant d'être éliminées, toutes les chutes doivent être retraitées selon le niveau de toxicité des composés ou principes actifs utilisés. Lors de cette étape, il est fréquent de découper la bande de film en plusieurs bandelettes de film de la largeur finale souhaitée, de regrouper ces bandelettes de film avant de les découper à la longueur voulue. A la fin de la découpe, les films sont donc regroupés en tas.

Les films obtenus sont ensuite stockés dans l'attente de leur conditionnement.

Le conditionnement est une étape cruciale de ce procédé puisqu'il consiste en une séparation d'un nombre déterminé de films. L'étape est délicate en ce qu'elle consiste à séparer des films de quelques microns d'épaisseur sans les scinder ou les dégrader.

Bien que dans le domaine alimentaire, le conditionnement multidoses est très répandu, dans le domaine pharmaceutique, de manière générale, un conditionnement définit une dose pharmaceutiquement active. En conséquence, cette définition du nombre de films par conditionnement est primordiale puisqu'il s'agit de produits qui, selon la dose ingérée, s'avéreront soit dangereux, soit bénéfiques. Les conditionnements sont généralement prévus pour un seul film.

La notion de dosage par film revêt de ce fait une importance capitale. Or, la dose de principe actif contenue dans un film obtenu par ce procédé de l'art antérieur dépend aussi bien, (i) du mélange effectué en début de fabrication, (ii) de la maîtrise de l'épaisseur de la bande de film au cours de l'étape d'étalement, (iii) de la surface de la bandelette de film puis du film découpé que (iv) de l'intégrité du film individualisé et conditionné. En conséquence, la garantie d'une dose donnée par conditionnement nécessite de nombreux points de contrôle à chaque étape de la chaîne de fabrication.

Les étapes des procédés de l'art antérieur sont généralement effectuées par des machines indépendantes les unes des autres et nécessitent donc d'une part, le transport des bobines de film, puis des bandelettes de film d'un appareil à l'autre mais aussi et surtout un contrôle des conditions ambiantes puisque les bobines sont stockées entre chaque étape du procédé. Les films solubles dans l'eau comme notamment les films oraux sont très sensibles à l'humidité ambiante. De fait, toute variation du taux d'humidité ambiante lors de leur fabrication, peut entrainer l'agglomération des bandes de film les unes aux autres et une perte de toute la production. De même, en conditions sèches, l'eau ayant un rôle de plastifiant, les films peuvent être fragilisés. Ainsi, la réduction de plasticité des films rend toute manipulation d'autant plus délicate avec des risques de rupture du film pouvant entraîner l'arrêt de la chaîne de production.

Par ailleurs, les nombreuses manipulations que ce soit, l'enroulement et le déroulement des bobines ou la séparation des bandes de film de leur support, les coupes des bandes en bandelettes puis des bandelettes en films et enfin la séparation des films obtenus au cours du conditionnement, sont source de nombreuses pertes le long de la chaîne de fabrication.

Des procédés similaires sont décrits dans l'art antérieur notamment dans la demande de brevet US 2004/0076799.

Dans le document JP 2004 231 516 A, un procédé est divulgué qui comporte les étapes suivantes:
a) préparation d'un mélange filmogène comprenant au moins un agent filmogène,
b) dépôt d'une dose dudit mélange dans une alvéole d'un support alvéolaire,
c) durcissement dudit mélange de sorte à obtenir un film,
d) obturation de ladite alvéole par un moyen d'obturation hermétique.

La présente invention permet de répondre aux différents problèmes de l'état de la technique en proposant un procédé en flux continu permettant l'obtention de films micro-dosés en conditionnement individuels. Le procédé selon l'invention comporte les étapes suivantes :
a) préparation d'un mélange filmogène comprenant au moins un agent filmogène,
b) dépôt d'une dose dudit mélange dans une alvéole d'un support alvéolaire, le mélange ayant une viscosité de 1 à 8000 mPa.s à la température dudit mélange filmogène lors de ladite étape b) de dépôt, préférentiellement une viscosité de 10 à 5500 mPa.s,
c) durcissement dudit mélange de sorte à obtenir un film,
d) obturation de ladite alvéole par un moyen d'obturation hermétique.

Les difficultés liées aux nombreuses manipulations des bandes et des bandelettes de films ou des films dans le procédé de l'art antérieur, source de pertes ou d'erreurs de dosage, ne sont pas observées dans le procédé selon l'invention. En effet, le film est directement obtenu dans son futur conditionnement. De même, en l'absence de découpe, il n'y a aucune perte de produits ou de principe actif. De plus, le film peut être de la forme et de l'épaisseur souhaitées suivant l'alvéole choisie et la quantité du mélange introduit dans cette alvéole.

Au sens de « film », on entend un produit mince et plan, de surface essentiellement plane, d'épaisseur maximale de 3 000 microns dans lequel l'épaisseur est très petite par rapport à la longueur et à la largeur. Le film au sens de la présente invention a de préférence une épaisseur comprise entre 10 et 3 000 microns, de préférence entre 20 et 2 000 microns.

Par ailleurs, il n'y a pas de problèmes de stockage des produits en cours de la chaîne de fabrication puisque le procédé s'effectue en continu. En outre, une fois le film obtenu dans l'alvéole, celle-ci est obturée hermétiquement par scellage par exemple et peut donc faire l'objet d'un stockage sans précautions particulières.

L'absence totale de manipulations, de pertes en cours de chaîne et de stockage de produits intermédiaires, réduit considérablement le coût de fabrication, que ce soit dans le cas d'une utilisation de produits présentant un danger pour les manipulateurs ou pour l'environnement, ou dans le cas de produits non recyclables ou onéreux. De fait, le volume d'air à traiter le long de la chaîne de fabrication est considérablement réduit.

En outre, le dosage par conditionnement est sûr puisque la dose introduite dans le conditionnement est finement déterminée et déterminable tout au long de la chaîne de fabrication.

Le procédé selon l'invention permet donc une réduction du coût de production, une augmentation du niveau de précision et de maîtrise des dosages ainsi que de la reproductibilité tout en étant adaptable à la fabrication de nombreux types de films tels que des films libres comme des films oraux ou des films d'arôme, des films comportant un support comme les patchs notamment.

Les difficultés résolues par la demanderesse sont d'une part, la réalisation d'un mélange filmogène permettant de par sa viscosité, un bon écoulement au moment du remplissage de l'alvéole et un bon étalement afin de former un film. Par agent ou substance filmogène, on entend toute substance susceptible de former un film.

Les procédés de l'art antérieur répondent à des contraintes différentes et utilisent de ce fait des mélanges filmogènes avec des caractéristiques physicochimiques différentes. En effet, ces procédés imposent une viscosité élevée et précise pour chaque mélange. A un niveau de viscosité légèrement inférieur, la composition filmogène s'écoule en dehors du support. Et à un niveau de viscosité légèrement supérieur, l'épaisseur de la bande de film n'est pas constante.

L'épaisseur de la couche destinée à former le film après durcissement peut être de 0,01 à 10 mm suivant la nature du mélange filmogène et l'application du film et donc suivant la perte de volume associée au durcissement du mélange donc à l'obtention du film.

Le procédé selon l'invention est rendu possible par un mélange ayant lors de son dépôt, une viscosité allant de 1 à 8000 mPa.s et préférentiellement 10 à 5500 mPa.s à la température du mélange au moment du dépôt et encore plus préférentiellement de 50 à 1500 mPa.s. Ce procédé est particulièrement avantageux en ce qu'il permet l'utilisation de mélanges de viscosités variées pour la production des films.

La viscosité souhaitée est atteinte dans le cas d'un mélange filmogène à base d'amidon à des températures allant de 10 à 95°C. Pour les polyéthylèneglycols de haut poids moléculaire, elle est atteinte à des températures supérieures environ à 50°C, de préférence entre 60 à 90°C.

La viscosité au sens de la présente invention est une viscosité Brookfield déterminée au moyen par exemple d'un viscosimètre Brookfield RDVD-I+ (Brookfield Engineering Laboratories, INC., Middleboro, MA, USA) en utilisant une des broches référencées RV1, RV2, RV3, RV4, RV5, RV6 ou RV7 et sans utilisation de l'équipement appelé "Helipath Stand". La rotation de la broche est fixée à 20 tours par minutes. La broche, de RV1 à RV7, est choisie de manière à ce que la valeur de viscosité affichée soit comprise entre 10% et 100% de l'échelle totale de viscosité possible avec la dite broche, comme indiquée par le constructeur. Pour effectuer cette mesure de viscosité, 300 ml du mélange filmogène sont placés dans un bécher de 400 ml de forme basse (diamètre environ 7,5 cm). La valeur de viscosité est prise à la fin de la 3^{ème} rotation. La mesure sera effectuée en suivant toutes les recommandations données par ce constructeur pour obtenir une mesure de viscosité fiable, par exemple dans le manuel "Operating Instructions, Manual N° M/92-021-M0101, Brookfield Digital Viscometer, Model DV-I+).

Selon une particularité de l'invention, les étapes b) et c) du procédé sont répétées au moins une fois avant l'étape d). Ce procédé étant adaptable à des mélanges aqueux et/ou organiques avec des agents filmogènes de natures différentes, les principes actifs ou arômes pouvant être utilisés sont très variés. Ainsi, par une répétition des étapes b et c), le procédé selon l'invention permet l'obtention d'un seul et même film à partir de produits ou principes actifs non miscibles entre eux ou incompatibles en mélange. En effet, dans ce cas, le film est fabriqué en deux fois, à partir de deux mélanges filmogènes de caractéristiques différentes.

Selon une autre variante avantageuse, le procédé selon l'invention prévoit une étape de dépôt dans ladite alvéole d'un composé granulaire ou solide ou d'une préparation semi-liquide ou liquide avant l'étape d). Autrement dit, le dépôt de composés granulaires, solides ou liquides peut se faire avant ou après l'étape b) voire après l'étape c). Ainsi, un composé ou une composition liquide ou semi-liquide peut être inclus(e) entre les deux couches de film dans le cas où les étapes b et c sont répétées. Par ailleurs, dans le cas d'un dépôt du mélange filmogène sur un support détachable tel qu'une feuille souple liée de façon réversible au fond de ladite alvéole, le composé ou la composition peut être inclus(e) entre la couche de mélange filmogène et la feuille souple. Dans les deux cas précités, le composé ou la composition peut être libéré(e) de façon contrôlée à la fonte de la ou des couche(s) de film. Le cas particulier d'un dépôt d'une substance semi-liquide ou liquide voire granulaire ou solide peut être l'impression du film ou d'un support contenu dans l'alvéole.

Ainsi, selon une variante avantageuse, ladite étape de dépôt d'un composé granulaire ou solide ou d'une préparation semi-liquide ou liquide est une étape d'impression. Ainsi, le film peut être imprimé afin d'indiquer le produit contenu, le dosage en toute lettre ou sous la forme de symbole avant l'obturation de ladite alvéole. L'impression d'une image, d'une photo ou d'un dessin dans le cas d'un film non destiné à une application pharmaceutique est envisageable.

De façon avantageuse, ladite étape d'impression est effectuée avant l'étape c) de durcissement dudit mélange. Ainsi, suivant l'effet souhaité et le système d'impression choisi, l'impression peut être envisagée sur le mélange non durci ou sur le film après l'étape c) de durcissement du mélange.

De façon encore plus avantageuse, ladite étape d'impression est effectuée avant l'étape b) de dépôt. En effet, dans le cas d'un film associé à un support, l'impression peut être envisagée de façon alternative entre le support et le mélange ou sur le mélange après dépôt.

L'impression peut être effectuée par une imprimante à jet d'encre ou tout autre moyen permettant une projection d'une composition colorée ou comportant une texture ou une opacité distincte dudit mélange filmogène de sorte à permettre l'apposition d'un motif, d'une image, d'une photo ou d'un texte sur ledit film.

Avantageusement, l'étape c) de durcissement du procédé selon l'invention est une étape de refroidissement du mélange à température ambiante. Par durcissement du mélange, on entend le passage de ce mélange d'une phase liquide ou visqueuse à une phase solide. Par exemple, lors de l'utilisation d'agents filmogènes qui fondent sous l'action de la chaleur et deviennent liquides sans incorporation de solvants. Tel est le cas notamment des polyéthylènes glycols (PEG) de haut poids moléculaire et de leurs dérivés, de polyéthylène oxydes et de leurs dérivés, de glycérides, poly glycérides et de leurs dérivés.

Selon les caractéristiques physicochimiques du mélange filmogène et notamment la volatilité du solvant dudit mélange filmogène, le procédé prévoit que l'étape c) de durcissement est une étape d'évaporation d'un solvant contenu dans ledit mélange filmogène.

Le procédé étant applicable à tous types de mélange filmogène, dans le cas d'un mélange organique dans lequel le solvant est une solution hydro-alcoolique comportant de l'éthanol, du propanol de l'isopropanol, ou tout autre solvant volatil, la simple évaporation du solvant à température ambiante suffit à son durcissement. Tel est le cas lorsque l'agent filmogène est un dérivé de cellulose telle que l'hydroxypropylcellulose, l'éthylcellulose, la méthylcellulose, la cellulose acétate phtalate, la cellulose acétate, un poly méthacrylate, un poly vinyle acétate phtalate.

Lorsque l'agent filmogène est un polymère hydrophile, l'eau sert de solvant, le polymère pouvant être un polymère d'origine végétale ou de synthèse, seul ou en mélange, par exemple et sans que cette liste soit limitative, les extraits d'algues tels que les alginates et les carraghénanes, les celluloses modifiées, les polysaccharides tels que le pullulan, la gélatine, la pectine, les gommes d'origine végétale ou animale, les polyéthylènes glycols, les poloxamers, les amidons natifs, les amidons modifiés et/ou hydrolysés, les maltodextrines, l'alcool polyvinylique et ses dérivés, ...

Avantageusement, l'étape d'évaporation du solvant s'effectue par augmentation de la température du mélange, par variation de pression ou par leur combinaison. L'étape d'évaporation du solvant peut s'effectuer à température réduite et à pression réduite de sorte à éviter des phénomènes de sublimation d'actifs ou pour ménager leur stabilité dans le cas d'une utilisation de composés labiles ou très réactifs à la chaleur. Les moyens d'échauffement du mélange pourront être l'étuvage du mélange ou le chauffage par micro-ondes ou par infrarouge ou la combinaison de ces moyens.

L'étape c) de durcissement est cruciale puisque le séchage du film doit être contrôlé, c'est-à-dire jusqu'à une teneur en solvant lui conférant tout à la fois une certaine structure, une certaine flexibilité et une stabilité à une humidité relative d'équilibre comprise entre 20 et 80%. Ainsi, dans le cas d'un solvant aqueux, la teneur en eau du film sera dans la majorité des cas, inférieure à 20% préférentiellement de l'ordre de 5 à 10% pour permettre une bonne conservation du film sans entraîner de craquellement de celui-ci. En effet, la structure filmogène doit être conservée mais le séchage doit s'effectuer préférentiellement sans collage du film dans l'alvéole.

Préférentiellement, le mélange filmogène comprend :
- de 40 à 99% en poids d'agent filmogène,
- au moins 1% en poids d'un additif.

Par additif, on entend un arôme, un édulcorant, un plastifiant, un humectant, un tensio-actif, un principe actif ou toute molécule pour laquelle l'agent filmogène est susceptible de constituer un vecteur.

De façon préférentielle, le mélange filmogène comprend un solvant aqueux ou organique. Avantageusement, l'agent filmogène comprend un amidon ou un mélange d'amidons.

De façon plus préférentielle, l'amidon ou au moins un amidon du mélange d'amidons est issu d'une légumineuse.

Le procédé est particulièrement adapté aux mélanges filmogènes obtenus à partir de mélanges d'amidons de diverses origines de manière à régler la teneur en amylose entre 25 et 45%. Par exemple des mélanges d'amidons riches en amylopectine (dits « waxy ») et d'amidons riches en amylose. L'amidon ou au moins un amidon du mélange peut notamment avoir subi au moins un traitement de modification choisi dans le groupe comprenant les traitements chimiques, les traitements physiques et les traitements enzymatiques.

Les traitements chimiques comprennent en particulier toutes les opérations connues d'estérification, d'éthérification, de réticulation ou d'hydrolyse par voies acide, oxydante ou enzymatique. Les traitements chimiques convenant particulièrement bien à l'obtention d'une solution filmogène sont les traitements dits de stabilisation que sont l'hydroxypropylation, l'acétylation, ces traitements pouvant éventuellement être complétés par une fluidification ou une hydrolyse ménagée, par exemple par traitement acide.

Le procédé est plus particulièrement adapté aux mélanges filmogènes obtenus à partir d'amidon de légumineuses. Par légumineuses on entend les plantes appartenant aux familles des césalpiniacées, des mimosacées ou des papilionacées et notamment toute plante appartenant à la famille des papilionacées comme, par exemple, le pois, le haricot, la fève, la fèverole, la lentille, la luzerne, le trèfle ou le lupin.

Cette définition inclut notamment toutes les plantes décrites dans l'un quelconque des tableaux contenus dans l'article de R. HOOVER et al. intitulé « Composition, structure, functionality and chemical modification of legume starches : a review ».

Avantageusement, il s'agit de pois, le terme « pois » étant ici considéré dans son acception la plus large et incluant en particulier :
- toutes les variétés sauvages de « pois lisse » (« smooth pea »), et
- toutes les variétés mutantes de « pois lisse » et de « pois ridé » (« wrinkled pea ») et ce, quelles que soient les utilisations auxquelles on destine généralement lesdites variétés (alimentation humaine, nutrition animale et/ou autres utilisations).

Lesdites variétés mutantes sont notamment celles dénommées « mutants r », « mutants rb », « mutants rug 3 », « mutants rug 4 », « mutants rug 5 » et « mutants lam » tels que décrits dans l'article de C-L HEYDLEY et al. intitulé « Developing novel pea starches » Proceedings of the Symposium of the Industrial Biochemistry and Biotechnology Group of the Biochemical Society, 1996, pp. 77-87.

Par « amidon de légumineuse », on entend toute composition extraite et ce, de quelque manière que ce soit, d'une légumineuse et notamment d'une papilionacée, et dont la teneur en amidon est supérieure à 40 %, de préférence supérieure à 50 % et encore plus préférentiellement supérieure à 75 %, ces pourcentages étant exprimés en poids sec par rapport aux poids sec de ladite composition.

Le procédé est applicable aux films de 10 à 3000 µm d'épaisseur, préférentiellement de 20 à 2000 µm, plus préférentiellement de 30 à 500 µm et encore plus préférentiellement de 35 à 200 µm, aussi bien dans le cas de films obtenus par un dépôt direct du mélange sur le fond de l'alvéole que sur un support maintenu au fond de l'alvéole. Dès lors, ce procédé est applicable à la fabrication de films que ce soit des films alimentaires ou pharmaceutiques, aux films oraux mais aussi aux films avec support tels que les patchs transdermiques permettant l'administration d'un principe actif par application du patch sur la peau ou toute autre application dans laquelle l'obtention d'un film d'une telle épaisseur est avantageuse. Par exemple, des films pharmaceutiques topiques, des films ou patch pharmaceutiques ou cosmétiques à apposer sur une muqueuse, sur une plaie ou sur la peau tels quels, ou à ajouter à un autre produit cosmétique avant utilisation, des films alimentaires consommés tels quels ou qui seront déposés sur un aliment avant consommation. Le procédé est aussi particulièrement adapté à la réalisation de films très solubles dans l'eau, films qui pourront être dissous dans de l'eau ou toute autre boisson avant consommation.

Après durcissement du film, suivant l'agent filmogène choisi, suivant le solvant utilisé, l'actif pourra se présenter sous forme d'une dispersion solide, sous forme cristalline et/ou amorphe. Ceci présente l'avantage de permettre une modulation de la solubilisation de cet actif.

L'additif peut être un arôme, un principe actif, un excipient, un complément alimentaire ou vitaminique, un extrait médicinal, un actif cosmétique, un colorant, un pigment, un correcteur de pH, un extrait végétal, un actif vétérinaire, des compléments alimentaires

Les principes actifs envisageables sont ceux dont l'application peut être transdermique tels que notamment les antifongiques, les antimycosiques, les corticoïdes, les anti-acnéiques, les antibiotiques, les antiparasitaires, les antihistaminiques, les keratolytiques, les antiseptiques, les molécules à visée répulsive, les rubéfiants, les dépigmentant, les anti-inflammatoires, les antirhumatismaux, les analgésiques, les topiques en phlébologie, les actifs anti-surcharge adipeuse, les contraceptifs, régulateurs de croissance; les hormones, anti-nauséeux...

Ou des principes actifs à application orale pour une action topique ou systémique tels que les antiseptiques, les antifongiques, les antimycosiques, les antibiotiques, les antihistaminiques, les anti-inflammatoires, les antimigraineux, les anti-diarrhéiques, les antalgiques, les antidépresseurs, les anesthésiques locaux, les antiparasitaires, les antipyrétiques, les antispasmodiques, ou toute autre substance présentant des propriétés curatives ou préventives à l'égard de maladies humaines ou animales ainsi que tout produit pouvant être administré à l'homme ou a l'animal en vue d'établir un diagnostique médical tel qu'une molécule agissant comme marqueur ou traceur, ou de restaurer, corriger ou modifier leurs fonctions organiques.

Nécessairement, les mélanges filmogènes pouvant être de nature variée, le matériau du support alvéolaire choisi devra être inerte vis-à-vis du mélange filmogène et notamment vis-à-vis du ou des composés contenus dans ce mélange.

De façon avantageuse, le procédé selon l'invention comporte une étape de réalisation dudit support alvéolaire.

Le support alvéolaire étant réalisable en fonction du film désiré, ce procédé peut être utilisé pour des films de toute forme et de toute taille.

Le support alvéolaire selon l'invention, est spécialement adapté à la mise en oeuvre du procédé selon l'invention et est notamment spécialement destiné à contenir un film d'une épaisseur de 10 à 3000 µm ou une structure pouvant atteindre 15 000 µm.

Également un dispositif comprenant ledit support alvéolaire est décrit.

Ce dispositif comporte un support alvéolaire pourvu d'une alvéole et d'un moyen d'obturation dudit support alvéolaire, ladite alvéole étant entourée d'une collerette transversale comprenant une zone de scellage hermétique, ladite alvéole étant pourvue d'un fond essentiellement plat, ledit fond (4b) ayant une surface supérieure à 1 cm² par millimètre de profondeur de ladite alvéole (4). Ce ratio permet une accessibilité aisée du film et facilite donc sa préhension. L'alvéole selon l'invention permet à la fois d'avoir une profondeur suffisante pour recevoir la composition filmogène avant l'étape de durcissement du mélange et une profondeur suffisamment faible pour faciliter la préhension du film sans pour autant le dégrader ou le fragiliser. En effet, le volume de la composition filmogène avant durcissement peut être beaucoup plus important que le volume du film obtenu du fait de l'évaporation du solvant ou de la polymérisation de l'agent filmogène. Ainsi pour une surface du fond de l'alvéole de 6 cm², elle peut comporter une hauteur de 1 à 2 mm dans le cas d'un film orodispersible. Dans le cas d'un patch, pour une surface de 64 cm² l'alvéole peut comporter une profondeur de 1 cm.

De façon avantageuse, ladite alvéole ayant une profondeur inférieure à 2 cm préférentiellement une profondeur de 0,02 à 1cm et plus préférentiellement de 0,05 mm à 10 mm, encore plus préférentiellement de 0,2 à 2 mm.

Par l'expression « essentiellement plat », on entend une structure ayant de manière générale une forme plate. Ainsi, le fond de l'alvéole peut être légèrement incurvé ou pourvu de micro-tubérosités ou protubérances, voir des microcavités ou de légères rainures. Une incurvation du fond de l'alvéole permet une augmentation de l'épaisseur du film et ainsi de la dose de principe actif ou de l'agent actif contenu dans un film tout en conservant la même surface et la même épaisseur en ses bords. Les micro-tubérosités ou microcavités ou micro-protubérances voire les légères rainures permettent elles, par la création d'une surface non homogène, d'éviter l'adhérence du film sur le fond de l'alvéole ou tout le moins de faciliter son décollement. Ces microcavités ou légères rainures peuvent être remplies d'infimes quantités de substances colorées, sous forme liquide ou de poudre, susceptibles d'adhérer à la composition filmogène après dépôt, de sorte à créer une structure formant un motif en surface du film en surépaisseur.

Avantageusement, l'alvéole est pourvue d'une zone de déformation. Selon une première variante préférentielle, la paroi latérale de l'alvéole est pourvue d'une zone de déformation. La zone de déformation occupe toute la hauteur de la paroi latérale ou une zone centrale ou périphérique de sa hauteur. Cette zone de déformation permet de réduire la hauteur de l'alvéole et ainsi d'améliorer l'accessibilité du film selon l'invention, par l'exercice d'une force sur le bord inférieur ou sur le fond de l'alvéole. Selon une seconde variante préférentielle, l'alvéole comprend une zone de déformation en son fond. Cette zone de déformation permet de faciliter la récupération du film. Avantageusement, la zone de déformation est localisée au niveau d'une bordure périphérique du fond de l'alvéole délimitant en son centre une zone de dépôt. De façon alternative, la zone de déformation s'étend sur la surface totale du fond de l'alvéole. La présence d'une bordure périphérique permet une délimitation de la périphérie du fond de l'alvéole et de réguler ainsi finement la contenance de la zone de dépôt.

Selon une variante avantageuse, le fond est pourvu d'une rainure périphérique. Cette rainure permet de rehausser le fond de l'alvéole vis-à-vis de la collerette et ainsi de faciliter l'accès au film tout en réduisant le volume de l'alvéole.

Les caractéristiques préférentielles du support alvéolaire sont les suivantes :
- profondeur inférieure à 2 cm, préférentiellement une profondeur de 0,02 à 1 cm et plus préférentiellement de 0,05 mm à 10 mm encore plus préférentiellement de 0,1 à 5 mm,
- la surface du fond de l'alvéole allant de 1 à 500 cm² préférentiellement de 2 à 250 cm² et plus préférentiellement de 3 à 125 cm².

Les matières thermoformées utilisées peuvent être du polychlorure de vinyle (PVC), du polyéthylène téréphtalate (PET), des polyamides (PA), du polychlorure de vinylidène (PVDC), du polystyréne (PS), du polypropylène, préférentiellement du PVC.

Les zones de déformations peuvent être constituées d'un des polymères précédemment cités ou de leur mélange ou de tout autre polymère lui conférant des caractéristiques d'élasticité.

Les alvéoles peuvent être obtenues également par formage par pression de l'aluminium ou d'un alliage comprenant de l'aluminium.

Avantageusement, l'alvéole du dispositif peut être pourvue d'un support détachable disposé au fond de l'alvéole et destiné à être recouvert au moins en partie par le film. Ledit support détachable peut être avantageusement lié de façon réversible au fond de ladite alvéole. Le support détachable est choisi parmi un textile tissé ou non tissé ou une feuille plastique ou métallique souple. Le produit obtenu peut être un élément inclus dans un film de façon transitoire (par destruction ou réduction du film par exemple) ou définitive, le film étant lui-même adhérent au support.

Selon une variante, ledit support détachable est pourvu sur au moins un de ses bords d'une zone d'adhérence destinée à être appliquée et à adhérer sur une surface. Dans ce cas, le produit obtenu peut être un film comportant un support tel qu'un patch. Ce dispositif permet donc d'avoir un patch directement conditionné obtenu en une seule étape sans risque de fragiliser la cohésion de l'ensemble mélange filmogène / support.

De façon avantageuse, l'invention porte sur un dispositif contenant un film. Par film on entend, un film libre, un film adhérant à un support ou un ensemble de plusieurs couches de mélanges filmogènes adhérant l'une à l'autre et formant un seul et même film.

L'invention a également pour objet un ensemble formé par l'assemblage de plusieurs dispositifs formant une plaquette, les dispositifs de la plaquette pouvant être séparés par au moins une ligne de séparation déchirable.

En outre, l'invention porte également sur une installation pour la réalisation du procédé comprenant la succession de moyens suivants :
- des moyens de fabrication et d'homogénéisation du mélange filmogène
- des moyens de dépôt du mélange filmogène,
- des moyens de durcissement du mélange
- des moyens de fermeture hermétique des alvéoles, et accessoirement
- des moyens d'impression.

Selon une variante, l'installation serait avantageusement pourvue de moyens de réalisation du dispositif spécialement destiné à la mise en oeuvre du procédé.

Le dispositif selon l'invention est plus amplement décrit et illustré ci-dessous.

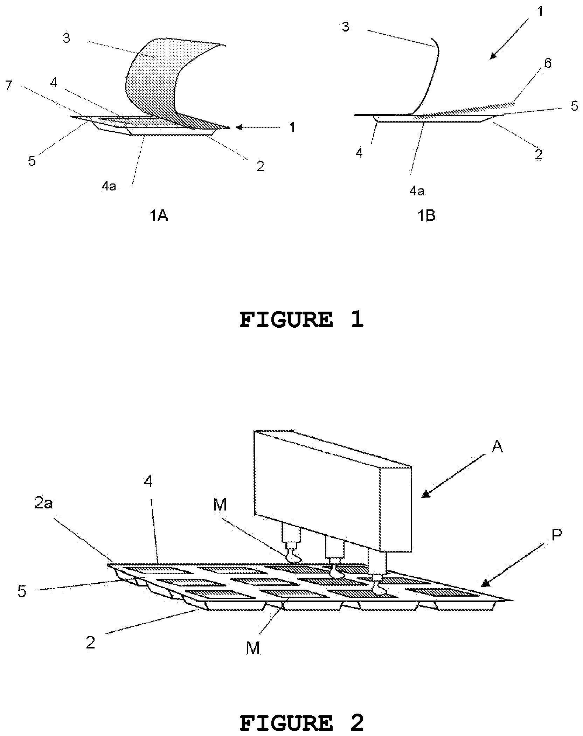

- Les figures 1A et 1B sont des vues d'une première variante du dispositif, la figure 1A est une vue en perspective de la face latérale de la première variante du dispositif composée d'un support alvéolaire et d'un feuillet d'obturation ; et la figure 1B est une vue latérale en coupe d'une première variante du dispositif contenant un film et dont le moyen d'obturation est partiellement ouvert ;

La figure 2 est une vue en perspective d'une portion d'une installation permettant le dépôt d'un mélange filmogène dans les alvéoles d'une plaquette de support alvéolaire ;

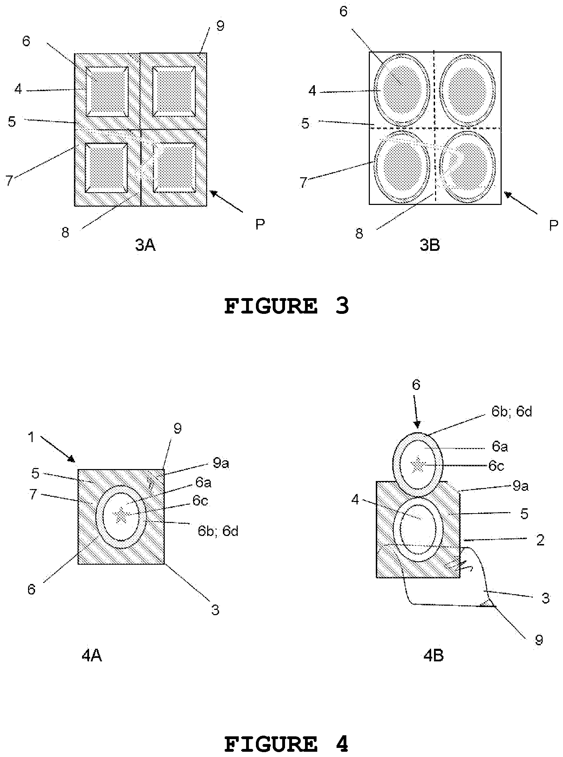

- Les figures 3A, 3B sont des vues du dessus de plaquettes contenant plusieurs dispositifs séparables, chaque alvéole de chaque dispositif contenant un film;
- Les figures 4A, 4B sont des vues du dessus de dispositifs contenant un patch, un dispositif fermé à la figure 4A et un dispositif ouvert avec le patch partiellement sorti de l'alvéole figure 4B ;

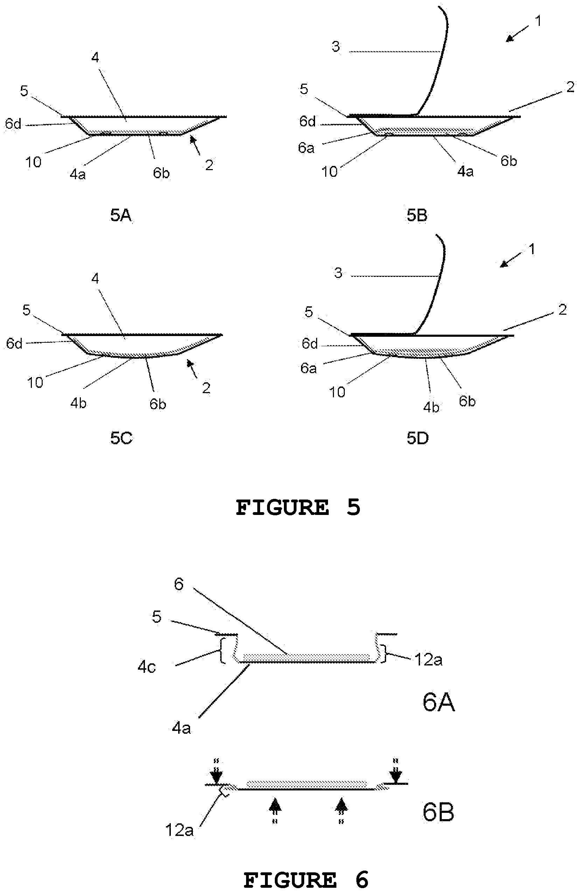

- La figure 5 illustre des vues latérales en coupe de variantes du dispositif selon l'invention, la figure 5A étant une vue d'une seconde variante du support alvéolaire vide et la figure 5B illustrant ce même dispositif partiellement ouvert et contenant un patch. Les figures 5C et 5D sont des vues latérales en coupe d'une troisième variante du dispositif selon l'invention, la figure 5C étant un vue du support alvéolaire vide et la figure 5D illustrant le dispositif partiellement ouvert et contenant un patch ;
- La figure 6 illustre des vues latérales en coupe d'une quatrième variante du dispositif selon l'invention comportant des zones de déformation localisées sur toute la hauteur de la paroi alvéolaire, la figure 6A étant un dispositif ouvert muni d'une zone de déformation dans la paroi alvéolaire dans une conformation non déformée et la figure 6B étant une vue du dispositif dans une conformation comprimée avec une déformation de la paroi latérale sous l'exercice de forces de compressions ;

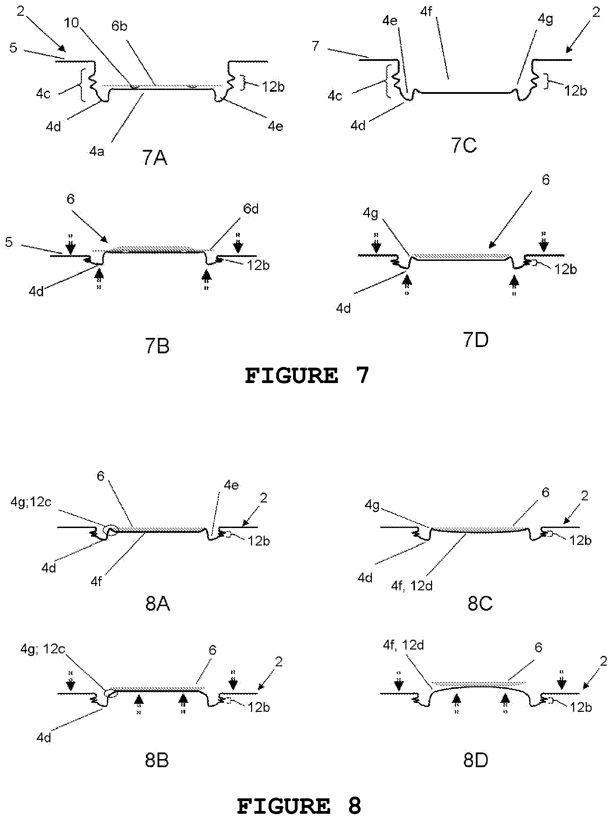

- La figure 7 illustre des vues en coupe des cinquième et sixième variantes du dispositif selon l'invention comportant des zones de déformation localisées dans une portion de la paroi alvéolaire, les figures 7A et 7C illustrant une vue d'un support alvéolaire vide et avant déformation ; les figures 7B et 7D illustrant le dispositif ouvert dans une conformation comprimée avec une déformation de la paroi alvéolaire et contenant un patch ou un film ;
- La figure 8 est composée des vues en coupe des septième et huitième variantes du dispositif ouvertes et contenant un film, ces dispositifs étant munis d'une zone de déformation dans la paroi alvéolaire dans une conformation comprimée et d'une zone de déformation dans le fond de l'alvéole, les figures 8A et 8C illustrant des dispositifs ayant la zone de déformation du fond de l'alvéole sous une conformation relâchée, les figures 8B et 8D illustrant les dispositifs ayant leurs zones de déformation au fond de l'alvéole dans une conformation déformée suite à l'exercice d'une force sur la base de l'alvéole.

La figure 1A illustre un dispositif 1 composé de deux structures, un support alvéolaire 2 et un moyen d'obturation sous la forme d'un feuillet 3 souple. Le support alvéolaire 2 est creusé d'une alvéole 4, le feuillet 3 étant soudé en sa périphérie à la zone de soudure 7 de la collerette 5 du support alvéolaire 2. Selon la première variante du dispositif, le fond 4a de l'alvéole 4 est plat.

Le moyen d'obturation 3 peut être un feuillet en aluminium, en plastique ou toute autre matière susceptible d'être collée ou scellée au support alvéolaire 2 de sorte à conférer une protection vis-à-vis de la lumière, de la chaleur, de l'humidité ou toute autre condition susceptible de nuire à la bonne conservation du contenu.

Le dispositif selon l'invention est spécialement destiné à contenir une structure essentiellement plane ayant une épaisseur de l'ordre de 10µm à 5000µm tel que notamment un film 6 (figure 1B). Le film 6 illustré dans la figure 1B est composé de deux structures différentes superposées formant un seul et même film. On peut ainsi envisager l'utilisation de mélanges filmogènes distincts avec deux principes actifs ou deux arômes distincts, voir un film incluant une substance liquide ou granulaire en son sein.

L'étape de dépôt du mélange M peut être effectuée par une large gamme de systèmes de distribution de doses A (figure 2) dans les alvéoles 4 d'une plaquette P. On peut citer notamment des appareils du type doseuse ou remplisseuse commercialisée par la société Industria Macchine Automatiche S.p.A. (I.M.A S.p.A.).

Le nombre de films fabriqués simultanément dépendra de la largeur des plaquettes P utilisables et donc de la capacité des systèmes A de dépôt de doses ainsi que des systèmes de durcissement du mélange.

Suite au dépôt, le mélange s'étale sur le fond 4a de l'alvéole 4. Cette étape d'étalement dépend de la viscosité du mélange qui peut être de 1 à 8000 mPa.s à la température du mélange filmogène lors de son dépôt. Elle dépend aussi de la tension de surface du mélange qui sera optimisée par l'ajout d'agents mouillant ou de tensio-actifs. Pour les viscosités les plus élevées, un système de vibration de la plaquette peut être envisagé pour faciliter l'étalement du mélange.

L'épaisseur de la couche destinée à former le film après durcissement peut être de 0,01 à 10 mm suivant la nature du mélange filmogène et l'application du film et donc suivant la perte de volume associée au durcissement du mélange donc à l'obtention du film.

Le choix de la technique permettant le durcissement du mélange de sorte à former le film dépend du mélange utilisé. Pour les mélanges sans solvant, le durcissement peut être envisagé simplement en laissant refroidir le mélange.

Dans le cas d'un mélange avec solvant volatil, le durcissement peut être réalisé par simple évaporation du solvant à température ambiante. Néanmoins, l'utilisation de chauffage et d'une réduction de pression permet d'accélérer le durcissement.

Dans le cas de mélanges aqueux, le choix de la technique de durcissement par évaporation de l'eau peut être varié, par exemple, par l'utilisation d'une étuve, de chauffage infrarouge ou de micro-ondes voire par réduction de pression.

La technique choisie doit permettre le maintien de la structure du film.

Après l'obtention du film, un nouveau cycle comportant les étapes b) et c) de dépôt et de durcissement est envisageable par exemple dans le cas d'intégration de deux principes actifs incompatibles, ou ayant des qualités physicochimiques distinctes de sorte qu'ils ne peuvent pas être intégrés dans le même mélange filmogène. La seconde couche du film étant appliquée sous forme liquide, adhère de ce fait à la première couche et confère par là même une unité de structure à l'ensemble.

L'obturation des alvéoles 4 peut s'effectuer par scellage ou collage sur une zone de scellage 7 d'un moyen d'obturation 3 pouvant être un feuillet souple ou rigide sur toute la surface des collerettes 5 séparant les alvéoles 4 (figure 3A) ou sur le pourtour de l'alvéole (figure 3B).

Les dispositifs peuvent être conditionnés sous forme de plaquettes P (figure 3) ou individuellement sous forme de dispositifs 1 (figure 4). Lorsqu'ils sont assemblés en plaquette P, les dispositifs 1 peuvent être individualisables par la formation de lignes de séparation déchirables 8 (figure 3).

Lorsque la zone de scellage 7 s'étend sur toute la collerette 5, une zone 9 de la collerette 5 est sécable de sorte à faciliter l'ouverture du dispositif 1 (figure 3A et figure 4).

La zone sécable 9 est obtenue par l'établissement d'une ligne de séparation déchirable 9a.

L'alvéole 4 des figures 4A et 4B contient un film avec support, est illustré à titre d'exemple, le cas d'un patch 6. Celui-ci est formé d'une zone filmogène 6a et d'un support 6b sous forme d'une feuille adhésive sensible à la pression.

Une impression 6c peut être envisagée sur le patch 6, de la même façon que sur un film sans support. Dans le cas des patchs, la zone d'impression 6c peut être effectuée sur le support 6b ou directement sur la zone filmogène 6a.

L'impression sur la zone filmogène peut être obtenue à partir d'une composition d'impression d'une coloration ou d'une texture différente de celle utilisée pour l'obtention de la zone filmogène. La composition d'impression peut être un simple colorant voir une seconde composition filmogène contenant une molécule non miscible dans la première composition filmogène. Ceci est transposable aux films sans support.

La seconde variante du dispositif est spécialement destinée à l'obtention d'un produit constitué d'un film sur un support tel est le cas des patchs par exemple. Cette variante contient avant coulage du mélange filmogène, un support 6b pouvant être un textile tissé, un textile non tissé ou une feuille plastique ou métallique souple ou non (figure 5). Une zone d'adhérence 6d peut être prévue sur une face du support détachable 6b correspondant à la face sur laquelle la zone filmogène 6a sera coulée, à savoir la face qui sera en contact avec la peau dans le cas d'un patch 6. Cette zone d'adhérence peut toutefois être constituée uniquement sur la périphérie du support 6b ou sur une portion de cette périphérie, l'adhérence étant obtenue par l'utilisation d'une colle sensible à la pression par exemple.

Le support 6b est plaqué au fond 4a, 4b de l'alvéole 4. Une zone d'adhésion 10 du support 6b au fond 4a, 4b de l'alvéole 4 peut être prévue de sorte que le support 6b soit faiblement fixé au fond 4a, 4b de l'alvéole 4 avant coulage de la zone filmogène 6a. Ainsi, à l'ouverture du dispositif, une simple traction sur le patch 6 permet sa libération et son utilisation.

Les étapes de durcissement et de scellage d'un moyen d'obturation 3 sont similaires aux variantes précédemment décrites.

En outre, la zone filmogène 6a peut être coulée en deux fois sur le support 6b adhésif sensible à la pression, lorsque deux principes actifs incompatibles en mélange doivent être introduits dans la même zone filmogène 6a ou lorsque la seconde couche confère l'adhésivité au support. Dans ce dernier cas, le second film peut être coulé sur toute la surface du support.

Le fond 4a, 4b de l'alvéole 4 selon l'invention est essentiellement plat c'est-à-dire qu'il peut contenir certaines irrégularités mineures ou de micro-irrégularités ou une faible incurvation ou inclinaison mais il conserve une structure majoritairement plane de sorte à permettre l'étalement du mélange et une épaisseur du film 6 relativement constante.

Ainsi, les figures 5A et 5B illustrent un dispositif ayant un fond 4a plat et les figures 5C et 5D illustrent un dispositif qui selon une troisième variante comporte un fond 4b de l'alvéole 4 légèrement incurvé. Dans le cas d'une variante comportant un support 6b, selon une première sous variante, le support 6b peut compenser le creux formé dans le fond 4b de l'alvéole 4, rétablissant pas là même un fond alvéolaire plat. On peut aussi envisager une seconde sous variante dans laquelle le support 6b peut reproduire le dénivelé (figure 5C). Dans ce dernier cas, la zone filmogène 6a obtenue comporte une partie localisée plus épaisse. Dans le cas illustré à la figure 5D cette localisation est centrale.

Selon ces variantes munies d'un support 6b au fond 4a, 4b de l'alvéole 4, on peut envisager avant dépôt du mélange filmogène, le dépôt d'une composition liquide ou semi-liquide ou le dépôt d'un composé granulaire ou solide directement sur le support 6b. Ainsi, la composition ou le composé serait inclus(e) entre les deux surfaces : la zone filmogène 6a d'un coté et le support6b de l'autre ; et la fonte ou la simple mise en contacte de la zone filmogène 6a sur la peau permettrait la libération contrôlée du composé ou de la composition ou l'adhérence du patch. Un résultat similaire peut être obtenu en incluant la composition ou le composé entre deux couches de mélanges filmogènes d'un film.

Selon des variantes préférentielles, la paroi alvéolaire 4c reliant le fond de l'alvéole 4a à la collerette 5 comporte une zone déformable 12a, 12b et un bord inférieur 4d. Cette zone déformable se déforme par pliure 12a ou torsion 12b de sorte qu'une pression (matérialisée dans les figures 6 et 7 par des doubles flèches) exercée sur le fond 4a (figure 6) ou sur le bord inférieur 4d de la paroi alvéolaire (figure 6) entraine une déformation de la paroi alvéolaire 4c permettant un rehaussement du fond 4a de l'alvéole au niveau de la collerette 5 de sorte à faciliter la préhension du film ou du patch (figure 6 et 7).

La zone déformable 12a et 12b peut s'étendre sur une portion de la paroi latérale (figure 7) ou sur la totalité de la paroi (figure 6).

Selon une variante avantageuse, le fond 4a comporte une rainure périphérique 4e permettant une surélévation du fond 4a par rapport au bord inférieur 4d de la paroi latérale 4c (figure 7). Cette surélévation du fond 4a facilite l'accessibilité du film lors de la déformation de la paroi alvéolaire 4c par pression sur le bord inférieur 4d. La partie centrale du fond 4a peut être plate (figure 7 A) ou une zone de dépôt 4f peut y être aménagée (figure 7B). La zone de dépôt 4f entourée d'une bordure 4g permet de ménager un très faible volume destiné à recevoir une composition filmogène pour le coulage du film.

Selon une autre variante (voir figures 8A et 8B), la bordure 4g constitue une nouvelle zone de déformation 12c permettant l'expulsion du film de la zone de dépôt par l'exercice d'une pression sur le fond de la zone de dépôt 4f. Alternativement selon une autre variante, la zone de dépôt 4f constitue dans sa totalité une zone de déformation 12d (figure 8B) et permet l'expulsion du film. En l'absence de bordure périphérique 4g et donc de la délimitation d'une zone de dépôt 4f, la zone de déformation 12d peut s'étendre sur tout le fond 4a, 4b de l'alvéole 4. Ces deux variantes sont transposables aux films accolés à un support.

Il faut noter que la zone de déformation 12c 12d située sur le fond 4a, 4b de l'alvéole 4 peut être prévue indépendamment des zones de déformations 12a, 12b des parois alvéolaires ou de la rainure périphérique 4e. Cependant, l'association d'une première zone de déformation 12a, 12b au niveau de la paroi alvéolaire, d'une rainure périphérique 4e et d'une zone de déformation 12a, 12b en bordure 4g ou dans la zone de dépôt 4f du fond 4a permet une accessibilité très facile des films ou patchs obtenus selon l'invention.

Les conditions de mise en oeuvre du procédé selon l'invention et des exemples non exhaustifs de compositions seront ci-dessous explicités.

### Exemples de films orodispersibles contenant de l'amidon comme agent filmogène

### Exemple 1 : Films rafraichissants

### - Obtention des mélanges :

La composition et les proportions sont détaillées dans le tableau 1. De l'eau est mélangée avec un plastifiant à savoir le glycérol (mélange E1), le dibutyl sébaçate (mélanges E2 et E4) ou le propylène glycol/acide oléique 25/75 (mélanges E3 et E5). Le colorant et le saccharinate sont ajoutés aux mélanges et homogénéisés pendant 5mn. Un amidon de pois fluidifié stabilisé vendu sous l'appellation LYCOAT® NG73 commercialisé par la demanderesse, est ensuite dispersé dans chaque solution obtenue de sorte à ce que les mélanges E1, E2, E3 soient à 12% d'amidon et les mélanges E4 et E5 à 6% d'amidon.

Chaque solution est mélangée pendant 5mn puis homogénéisée pendant 3mn puis chauffée jusqu'à ce qu'il atteigne la température de 70°C.

**Tableau 1**

| **Composition** | | **E1** | **E2** | **E3** | **E4** | **E5** |
|---|---|---|---|---|---|---|
| Amidon de pois fluidifié stabilisé (g) | | 60,20 | 57,5 | 57,5 | 28, 75 | 28, 75 |
| Plastifiant (g) | Glycérol | 8,9 | | | | |
| | Dibutyl sébaçate Fluka | | 8,9 | | 4, 45 | |
| | Propylène glycol/acide oléique 25/75 | | | 8,9 | | 4, 45 |
| Arôme (g) | Arôme menthe Silésia | 11,15 | 11,15 | 11,15 | 5,6 | 5,6 |
| | Menthol | 2,3 | 2,30 | 2,30 | 1,15 | 1,15 |
| Polysorbate (Tween 80) (g) | | 0,4 | 0,4 | 0,4 | 0,2 | 0,2 |
| Lécithine de soja (g) | | 0,4 | 0,4 | 0,4 | 0,2 | 0,2 |
| Saccharinate de sodium (g) | | 2,3 | 2,3 | 2,3 | 1,15 | 1,15 |
| Colorant bleu E133 Silésia 2%MS (g) | | 4,45 | 4,45 | 4,45 | 2,2 | 2,2 |
| Solvant (g) | Eau | 409,9 | 412,6 | 412,6 | 456,3 | 456,3 |
| | Ethanol | | | | | |
| Total (g) | | 500 | 500 | 500 | 500 | 500 |
| **Température de dépôt et de mesure de viscosité (°C)** | | 45 | 45 | 40 | 40 | 40 |
| **Viscosité (mPa.s)** | | 2500 | 2200 | 2530 | 250 | 270 |

Les mélanges sont ensuite refroidis à 40 ou 45°C et les arômes, le polysorbate et la lécithine de soja sont incorporés et mélangés sous vide pendant 10mn. Les mélanges sont maintenus à 45°C ou à 40°C en étuve jusqu'au remplissage des alvéoles.

Les viscosités des mélanges (voir tableau 1) sont mesurées avec l'appareil viscosimètre Brookfield RDVD-I+, avec la broche RV5 pour les mélanges E1, E2 et E3 et la broche RV3 pour les mélanges E4 et E5, à une vitesse de 20tr/mn, à la température de 40°C ou 45°C, température de dépôt choisie pour obtenir une viscosité idéale du mélange favorable à son étalement dans l'alvéole.

### - Variation de l'épaisseur des films en fonction du volume de dépôt :

Différents volumes du mélange sont déposés dans le fond des alvéoles de sorte à obtenir des films de différentes épaisseurs.

Des volumes de V1 V2, V3 sont coulés pour les mélanges E1, E2 et E3, tels que V1 = 0,4 cm³, V2 = 0, 3 cm³ et V3 = 0,2 cm³.

Les alvéoles sont obtenues à partir de feuilles de plastique thermoformable et ont les caractéristiques suivantes :
- profondeur de 5 mm
- surface du fond de l'alvéole 406 mm2.

Le séchage est effectué par sécheur micro-ondes de sorte à obtenir des films ayant une teneur en eau inférieure à 10%.

Le séchage par micro-ondes a été effectué au moyen d'un sécheur à micro-ondes Pulsar ST22 de la société Microondes Energie Systemes (MES) selon deux conditions différentes :
1. Vitesse du tapis : 5.25 m/mn
   Puissance micro-ondes : 100W pendant 47mn puis à 200W pendant 18mn
2 Vitesse du tapis : 5.25m/mn
   Puissance micro-ondes : 300W pendant 50mn

Les films obtenus avec les volumes déposés ont une épaisseur sensiblement égale pour les mélanges E1, E2 et E3 de l'ordre de 200 µm (V1), 150µm (V2) et 100µm (V3).

Ces conditions ont permis l'obtention de films non collés au fond de l'alvéole. Ces films orodispersibles ont de plus, de très bonnes qualités gustatives et une bonne texture en bouche.

### Variation des techniques de séchage :

Pour les mélanges E1, E2 et E3 des volumes de 0,3cm³ ont été déposés. Différents modes de séchage sont testés parmi ceux-ci séchage à l'étuve à 45°C et séchage par réduction de pression en établissant le vide et la combinaison de ces deux modes.
1/ séchage à l'étuve à 45°C durant 2 heures
2/ séchage à température ambiante par établissement du vide (Etuve sous vide 3608-Ic, LAB-LINE Instruments, Inc., Melrose Park, Ill., USA) durant 3 heures
3/ séchage à l'étuve à 45°C et par établissement du vide (Etuve sous vide 3608-Ic, LAB-LINE Instruments, Inc., Melrose Park, Ill., USA) durant 45 min.

Ces différents modes de séchage ont conduit à l'obtention de films de caractéristiques similaires à ceux obtenus par séchage micro-ondes à savoir des films orodispersibles de très bonnes structures et qualités organoleptiques.

La technique de combinaison de réduction de pression et de chauffage à 45°C permet une réduction importante de la durée de séchage et donc favorise la préservation des composés instables en solution aqueuse.

### Exemple 2 : Films orodispersibles avec principe actif

La composition E6 est obtenue par le mélange dans un premier temps, de l'eau, du glycérol, d'un colorant (E133 Silésia) et du saccharinate dans les proportions décrites dans le tableau 2. Le mélange est homogénéisé pendant 5mn. Un actif, le Meloxicam est ajouté en mélange avec l'amidon puis homogénéisé pendant 3mn. Le mélange est chauffé jusqu'à ce qu'il atteigne la température de 70°C.

Le mélange est ensuite refroidi à 45°C et les arômes, le polysorbate et la lécithine de soja sont incorporés et mélangés sous vide pendant 10mn. Le mélange est maintenu à 45°C en étuve jusqu'au remplissage des alvéoles.

**Tableau 2**

| **Composition** | | **E6** | **E7** |
|---|---|---|---|
| Amidon de pois fluidifié stabilisé (g) | | 28, 75 | 28, 75 |
| Plastifiant (g) | Glycérol | 4, 45 | |
| | Dibutyl sébaçate Fluka | | 4, 45 |
| Arôme (g) | Arôme menthe Silésia | 5,6 | 5,6 |
| | Menthol | 1,15 | 1,15 |
| Polysorbate (Tween 80) (g) | | 0,5 | 0,2 |
| Lécithine de soja (g) | | 0,5 | 0,2 |
| Actif (g) | Meloxicam | 12,5 | |
| | Lopéramide HCl | | 3,5 |
| Saccharinate de sodium (g) | | 1,15 | 1,15 |
| Colorant bleu E133 Silésia 2%MS (g) | | - | 2,2 |
| Solvant (g) | Eau | 345,4 | 452,8 |
| | Ethanol | 100 | |
| Total (g) | | 500 | 500 |
| **Température de dépôt et de mesure de viscosité** (°C) | | 45 | 40 |
| **Viscosité** (mPa.s) | | 280 | 240 |

Les viscosités des mélanges (voir tableau 2) sont mesurées avec l'appareil viscosimètre Brookfield RDVD-I+, avec la broche RV3 à une vitesse de 20tr/mn, à la température de 45°C, température de dépôt choisie pour obtenir une viscosité idéale du mélange favorable à son étalement dans l'alvéole.

L'obtention du mélange E7 s'effectue comme celui des mélanges E1 à E5, l'actif étant ajouté au mélange seul avant l'ajout de l'amidon puis homogénéisé pendant 5mn.

Un dépôt de 0,3cm³ des mélanges E6 et E7 est effectué par alvéole et l'ensemble est mis pour moitié en étuve à 45°C une nuit pour le séchage et pour moitié laissé à température ambiante une nuit.

Le séchage à température ambiante étant plus doux que celui en étuve à 45°C, des films ayant une plus haute humidité sont obtenus avec séchage à température ambiante donc des films moins cassants.

### Exemple de films orodispersibles contenant du Polyéthylène glycol comme agent filmogène (Exemple 3)

**Tableau 3**

| **Composition** | **E8** |
|---|---|
| Polyéthylène glycol 4000(g) | 392.5 |
| Dextrométhorphane HBR | 7,5 |
| **Viscosité à 60°C (mPa.s)** Broche RV3 | 550 |

La composition filmogène E8 est obtenue à partir de Polyéthylène glycol sans adjonction de solvant (voir tableau 3), le polyéthylèneglycol étant sous forme liquide par fusion à 60°C. Les mesures de viscosité (tableau 3) sont effectuée comme précédemment.

Des dépôts de 800 mg sont effectués dans chaque alvéole, ce qui correspond à un dosage unitaire de 15 mg de dextrométhorphane.

Les films sont durcis à température ambiante par refroidissement des dépôts. Des films orodispersibles de bonnes textures sont obtenus.

## Revendications

1. Procédé de fabrication d'un film (6) comportant les étapes suivantes :
a) préparation d'un mélange filmogène comprenant au moins un agent filmogène,
b) dépôt d'une dose dudit mélange dans une alvéole d'un support alvéolaire, le mélange ayant une viscosité de 1 à 8000 mPa.s à la température dudit mélange filmogène lors de l'étape b) de dépôt,
c) durcissement dudit mélange de sorte à obtenir un film (6),
d) obturation de ladite alvéole (4) par un moyen d'obturation (3) hermétique.

2. Procédé selon la revendication 1 dans lequel les étapes b) et c) sont répétées au moins une fois avant l'étape d).

3. Procédé selon l'une ou l'autre des revendications 1 ou 2, **caractérisé en ce qu'**une étape de dépôt d'un composé granulaire ou solide ou d'une préparation semi-liquide ou liquide est prévue dans ladite alvéole (4) avant l'étape d).

4. Procédé selon la revendication 3, **caractérisé en ce que** ladite étape de dépôt d'un composé granulaire ou solide ou d'une préparation semi-liquide ou liquide est une étape d'impression.

5. Procédé selon l'une quelconque des revendications 1 à 4, **caractérisé en ce que** l'étape c) de durcissement est une étape de refroidissement du mélange ou une étape d'évaporation d'un solvant contenu dans ledit mélange filmogène.

6. Procédé selon la revendication 5, **caractérisé en ce que** l'étape d'évaporation dudit solvant s'effectue par augmentation de la température du mélange, par variation de pression ou par leur combinaison.

7. Procédé selon l'une quelconque des revendications 1 à 6, **caractérisé en ce que** le mélange comprend :
- de 40 à 99% en poids d'agent filmogène,
- au moins 1% en poids d'un additif.

8. Procédé selon l'une quelconque des revendications 1 à 7, **caractérisé en ce que** l'agent filmogène comprend un amidon ou un mélange d'amidons.

9. Procédé selon l'une quelconque des revendications 1 à 8, **caractérisé en ce que** l'agent filmogène comprend un amidon de légumineuse préférentiellement de pois.

10. Procédé selon l'une quelconque des revendications 1 à 9, **caractérisé en ce que** le procédé comporte une étape de réalisation dudit support alvéolaire (2).

## Patentansprüche

1. Verfahren zur Herstellung eines Films (6), welches die folgenden Schritte umfasst:
a) Herstellung einer filmbildenden Mischung, welche wenigstens ein filmbildendes Mittel umfasst,
b) Einbringen einer Dosis dieser Mischung in einen Hohlraum eines Hohlraumträgers, wobei die Mischung bei der Temperatur der filmbildenden Mischung während Schritt b) des Einbringens eine Viskosität von 1 bis 8000 mPa.s aufweist,
c) Härtung der Mischung, um einen Film (6) zu erhalten,
d) Verschließen des Hohlraums (4) mittels eines hermetischen Verschlussmittels (3).

2. Verfahren nach Anspruch 1, wobei die Schritte b) und c) vor Schritt d) wenigstens einmal wiederholt werden.

3. Verfahren nach dem einen oder anderen von Anspruch 1 oder 2, **dadurch gekennzeichnet, dass** vor dem Schritt d) ein Schritt des Einbringens einer körnigen oder festen Verbindung oder einer halbflüssigen oder flüssigen Zubereitung in den Hohlraum (4) vorgesehen ist.

4. Verfahren nach Anspruch 3, **dadurch gekennzeichnet, dass** der Schritt des Einbringens einer körnigen oder festen Verbindung oder einer halbflüssigen oder flüssigen Zubereitung ein Druckschritt ist.

5. Verfahren nach einem der Ansprüche 1 bis 4, **dadurch gekennzeichnet, dass** der Schritt c) des Härtens ein Schritt des Abkühlens der Mischung oder ein Schritt des Verdampfens eines in der filmbildenden Mischung enthaltenen Lösungsmittels ist.

6. Verfahren nach Anspruch 5, **dadurch gekennzeichnet, dass** der Schritt des Verdampfens des Lösungsmittels durch Erhöhung der Temperatur der Mischung, durch Veränderung des Drucks oder durch deren Kombination erfolgt.

7. Verfahren nach einem der Ansprüche 1 bis 6, **dadurch gekennzeichnet, dass** die Mischung umfasst:
- 40 bis 99% Gewichtsprozent filmbildendes Mittel,
- wenigstens 1 Gewichtsprozent Zusatzstoff.

8. Verfahren nach einem der Ansprüche 1 bis 7, **dadurch gekennzeichnet, dass** das filmbildende Mittel eine Stärke oder eine Stärkemischung umfasst.

9. Verfahren nach einem der Ansprüche 1 bis 8, **dadurch gekennzeichnet, dass** das filmbildende Mittel eine Hülsenfruchtstärke, bevorzugt aus Erbsen, umfasst.

10. Verfahren nach einem der Ansprüche 1 bis 9, **dadurch gekennzeichnet, dass** das Verfahren einen Schritt der Ausführung des Hohlraumträgers (2) umfasst.

## Claims

1. A process for manufacturing a film (6) comprising the following steps:
a) preparing a film-forming mixture comprising at least one film-forming agent,
b) depositing a dose of said mixture into a cavity of a cavity support, the mixture having a viscosity of 1 to 8000 mPa.s at the temperature of said film-forming mixture during the deposition step b),
c) curing of said mixture so as to obtain a film (6),
d) closing said cavity (4) by a hermetic closure means (3).

2. The process as claimed in claim 1, wherein the steps b) and c) are repeated at least once before step d).

3. The process as claimed in one or other of claims 1 and 2, wherein a step of depositing a granular or solid compound or a semi-liquid or liquid preparation in said cavity (4) before step d) is provided.

4. The process as claimed in claim 3, wherein said step of depositing a granular or solid compound or a semi-liquid or liquid preparation is a printing step.

5. The process as claimed in any one of claims 1 to 4, wherein the curing step c) is a step of cooling the mixture or a step of evaporating a solvent contained in said film-forming mixture.

6. The process as claimed in claim 5, wherein the step of evaporating said solvent is carried out by increasing the temperature of the mixture, by varying the pressure or by a combination thereof.

7. The process as claimed in any one of claims 1 to 6, wherein the mixture comprises:
- from 40 to 99% by weight of film-forming agent,
- at least 1% by weight of an additive.

8. The process as claimed in any one of claims 1 to 7, wherein the film-forming agent comprises a starch or a mixture of starches.

9. The process as claimed in any one of claims 1 to 8, wherein the film-forming agent comprises a legume starch, preferably pea starch.

10. The process as claimed in any one of claims 1 to 9, wherein the process comprises a step of producing said cavity support (2).
